Europäisches Patentamt

European Patent Office

Office européen des brevets

(11) Publication number: **0 084 706**
**B1**

## (12) EUROPEAN PATENT SPECIFICATION

(45) Date of publication of patent specification: **12.02.86**

(21) Application number: **82306259.1**

(22) Date of filing: **24.11.82**

(51) Int. Cl.⁴: **B 01 J 27/18, B 01 J 37/16,**
**C 07 C 51/25, C 07 C 57/145,**
**B 01 J 27/198, B 01 J 27/28**

(54) Activation process for fluid bed oxidation catalysts useful in the preparation of maleic anhydride.

(30) Priority: **28.12.81 US 334693**

(43) Date of publication of application:
**03.08.83 Bulletin 83/31**

(45) Publication of the grant of the patent:
**12.02.86 Bulletin 86/07**

(84) Designated Contracting States:
**BE DE FR GB IT NL**

(56) References cited:
**BE-A- 850 222**
**FR-A-2 237 897**
**FR-A-2 297 081**
**US-A-3 899 516**
**US-A-4 100 106**
**US-A-4 122 096**
**US-A-4 152 339**
**US-A-4 171 316**
**US-A-4 178 298**
**US-A-4 181 628**
**US-A-4 252 635**

(73) Proprietor: **THE STANDARD OIL COMPANY**
**Midland Building**
**Cleveland, Ohio 44115 (US)**

(72) Inventor: **Blum, Patricia Rae**
**970 Brookpoint Drive**
**Macedonia Ohio 44056 (US)**
Inventor: **Nicholas, Mark Lee**
**12414 Mt. Overlook**
**Cleveland Ohio 44120 (US)**
Inventor: **Milberger, Ernest Carl**
**34765 Sherwood Drive**
**Solon Ohio 44139 (US)**
Inventor: **Zock, Patricia Ann**
**601-C Lee Road, No. 1130**
**Bedford Ohio 44146 (US)**

(74) Representative: **Smith, Sydney et al**
**Elkington and Fife High Holborn House 52/54**
**High Holborn**
**London WC1V 6SH (GB)**

Courier Press, Leamington Spa, England.

**Description**

The present invention relates to the activation of fluid bed oxidation catalysts. More particularly the present invention relates to the activation of fluid bed oxidation catalysts useful in the preparation of maleic anhydride from 4-carbon atom hydrocarbons, including n-butane.

Oxidation catalysts containing the mixed oxides of vanadium and phosphorus have been utilized to produce maleic anhydride from 4-carbon atom hydrocarbons, such as n-butane. Methods have been investigated for activating, or increasing the catalytic activity of these catalysts. It is taught in the literature to "condition" vanadium phosphate-containing maleic anhydride catalysts under the flow of a low level of hydrocarbon in air, such as 0.2 volume percent to 2 volume percent hydrocarbon in air at temperatures of 300°C to 600°C, as in U.S. patent No. 4,171,316.

U.S. Patent No. 4,122,096 teaches conditioning of a dehydrated catalyst precursor with CO, $H_2$ or $H_2S$ in the absence of oxygen, at a temperature of from 300°C to 600°C.

U.S. Patent Nos. 4,178,298 and 4,181,628 discuss activating a mixed vanadium and phosphorus oxide catalyst at temperature of 300°C to 500°C by passing a gaseous hydrocarbon component having 2 to 6 carbon atoms with the exclusion of molecular oxygen over the catalyst.

In FR—A—2,237,897 there is disclosed a process for the oxidation of alkanes to anhydrides of dicarboxylic acids in which catalysts containing vanadium and phosphorus are used. Reference is made to the fact that the catalyst may be conditioned by treatment with a mixture of hydrocarbon and air at a temperature of about 450°C before carrying out the oxidation itself. There is disclosure of passing *n*-butane and air over the catalyst at a rate respectively of 40 ml per minute and 1000 ml per minute. This corresponds to a butane/oxygen molar ratio of 1 to 5.2, so that the ratio of oxygen to butane is slightly less than the stoichiometric ratio required for total oxidation of the butane.

Attempting to "condition" a fluid bed catalyst with low level of hydrocarbon in air under normal operating conditions has been found to have little beneficial effect. Further, it is impractical and nearly impossible to utilize an in-reactor activation method in a fluid bed reactor which comprises contacting the fluidized catalyst with a hydrocarbon at high temperature in the absence of molecular oxygen. Commercial fluid bed reactors do not contain means for heating the catalyst bed and gas streams to the temperatures required to achieve the desired activation utilizing oxygen-free hydrocarbon feeds. The requisite external heating mode of operation is not desired for fluid bed reactions, and indeed fluid bed processes are attractive for the reason that such external heating means are not required for normal operation.

Where it is desired to activate catalysts containing the mixed oxides of vanadium and phosphorus for the partial oxidation of n-butane to form maleic anhydride, the activation of the catalyst with the hydrocarbon n-butane in the absence of oxygen would be extremely expensive, from the point of view of the volume of butane required to "fluidize" the catalyst bed. In addition, stringent safety precautions would also be required to insure that no explosive mixtures of air/butane result outside the reactor.

Apart from economic and mechanical considerations, it is thought that contacting the vanadium phosphorus mixed oxide catalyst with reducing gases, including hydrocarbons, in the absence of oxygen merely causes reduction of the catalyst components (particularly vanadium) by extracting lattice oxygen, causing the catalyst crystallite structure to reach a static configuration. Oxygen atoms are thus depleted from the catalytic active sites, and are unavailable for reaction with hydrocarbon reactants to yield useful product. While this procedure may enhance the activity of catalysts that were initially over-oxidized, the overall effect upon catalysts having a proper component valence range could be detrimental over a period of time by inducing change to the static configuration.

We have found that fluid bed maleic anhydride catalysts containing the mixed oxides of vanadium and phosphorus can be activated in situ (in the fluid bed reactor) by contacting the fluidized catalyst with oxygen and a reducing gas at least partially combustible with oxygen at an elevated temperature sufficient to cause such combustion wherein the molar ratio of the amount of reducing gas to oxygen is in a particular range as mentioned below.

Contacting the catalyst, while fluidized, with both oxygen and a reducing gas at least partially combustible with oxygen at an elevated temperature sufficient to cause such combustion surprisingly provides a more effective, dynamic activation of the catalyst. In contrast to the static extraction of oxygen from the catalyst to provide reduction as in the prior art, when oxygen is present together with the combustible reducing gas as in the process of the present invention, at elevated temperature, the catalyst is induced to operate catalytically, and a dynamic, interactive catalytic activation process results. The catalyst is permitted to take up oxygen as well as yield oxygen to the reducing feed component, allowing the active site and catalyst microcrystalline structure to undergo dynamic reorientation. This is thought to result in localised crystalline phase changes which optimize the catalyst activity.

Because the activation procedure is carried out at temperatures sufficient to cause at least partial combustion of the reducing gas, the required activation temperature is attained by the heat of combustion after initial startup heating by conventional fluid bed preheaters.

The fact that partial combustion takes place in the fluidized bed lessens the concentration of heated combustible reducing gas in the effluent stream, such reducing gas being diluted by the combustion products, although precautions should still be taken to insure that an explosive mixture is not permitted to develop downstream of the reactor. The presence of the combustion product diluents, such as $H_2O$ if $H_2$ is

utilized as the reducing gas, $H_2O$ and $SO_2$ if $H_2S$ is utilized as the reducing gas, and $H_2O$ and $CO_2$ if hydrocarbon is utilized as the reducing gas, decreases the concentration of combustible gas in the effluent. The activating feed of the present invention, utilizing air in addition to reducing gas, is also less costly.

The process of the present invention involves activating a fluid bed catalyst by contacting a fluidized catalyst containing the mixed oxides of vanadium and phosphorus with oxygen and a reducing gas at least partially combustible with oxygen at an elevated temperature sufficient to cause combustion, wherein the molar ratio of reducing gas to oxygen is above the stoichiometric ratio required for complete combustion of the reducing gas, characterized in that a ratio of reducing gas to oxygen from 10:1 to 1:3 is employed.

The present invention further provides a process for producing maleic anhydride, including a process step in which the catalyst or a part thereof is activated as described above.

Catalysts for the production of maleic anhydride from 4-carbon atom hydrocarbon, such as n-butane, butenes and butadiene, particularly n-butane, generally contain the mixed oxides of vanadium and phosphorus. The catalysts may additionally contain promoter elements, including but not limited to alkali or alkaline earth metals, titanium, zirconium, hafnium, niobium, molbydenum, iron, cobalt, nickel, copper, zinc, cadmium, rare earths, cerium, uranium and mixtures thereof. The molar ratio of promoter elements to vanadium is generally 0.001:1 to 1:1, preferably 0.1:1 to 0.5:1. The molar ratio of phosphorus to vanadium is generally 0.5:1 to 2:1, preferably 0.9:1 to 1.6:1. The valence of the vanadium component of the catalyst is generally reduced from the pentavalent state, the valence of vanadium generally being between 3.5 to 4.6 and preferably being approximately 4. The maleic anhydride catalyst may additionally contain diluents or supports, such as titania, alumina, alumina-silica, zirconia, silica, and silicon carbide.

The catalysts may be prepared by reacting catalyst component containing compounds in the presence or absence of a corrosive reducing agent in a liquid, including but not limited to water, alcohols, aldehydes, glycols, ketones and halogenated olefins. Suitable corrosive reducing agents to provide vanadium in the proper valence state include but are not limited to HCl, HBr, and oxilic acid. Suitable liquid media capable of reducing vanadium to its proper valence state include but are not limited to isopropanol, isobutanol, crotylalcohol, allyl alcohol, isopentanol, acetaldehyde, propionaldehyde, butyraldehyde, ethylene glycol, methyl ethyl ketone, perchloropropene and hexachlorobutadiene.

Suitable vanadium compounds for use in preparing tbe maleic anhydride catalysts include vanadium pentoxide or vanadium salts, such as ammonium metavanadate and vanadium oxytrihalides. Suitable phosphorus containing compounds include phosphoric acid, including metaphosphoric acid, orthophosphoric acid, triphosphoric acid and pyrophosphosphoric acid and phosphorus pentoxide, phosphorus oxyiodide, phosphorus oxychloride and phosphorus pentachloride. Suitable promotor elements containing compounds include promoter metal oxides, hydroxides, nitrates, halides or salts of organic acids such as acetates, formates, butyrates and benzylates.

The catalyst components are mixed in the liquid medium, before or after the vandium component is reduced to its proper valence state. The catalyst precursor formed is recovered and dried. The catalyst is formed into fluid bed form by crushing and screening the catalyst particles to a proper size, such as in the range of 20 to 300 microns, by the oil drop method, wherein an aqueous solution or slurry of the catalyst is dropped into a heated oil bath to form solid particles, or by spray drying to form the desired particles. The catalyst may be calcined before or after forming into the fluidizable particles, dependent upon the method of preparation chosen. A method of preparing fluidizable catalysts useful for the production of maleic anhydride from 4-carbon atom hydrocarbons such as n-butane is disclosed in EP—A 0056901 (Application No: 81305882.3). The specific method of preparing the catalysts to be activated is not, however, critical to the process of the present invention.

Hydrocarbons reacted to form maleic anhydride include n-butane, n-butenes, 1,3 butadiene, or a mixture thereof. The molecular oxygen used in the reaction is most conveniently added as air, but synthetic streams containing molecular oxygen are also suitable. In addition to the hydrocarbon and molecular oxygen, other gases may be added to the reactant feed, such as steam or nitrogen. Preferably, oxygen/hydrocarbon ratios in the reactor feed are from 4 to 20 moles of oxygen per mole of hydrocarbon.

The reaction temperature may vary widely and is dependent upon the particular hydrocarbon and catalyst employed. Temperatures of 325°C to 500°C are preferred. The reaction may be conducted at atmospheric, superatmospheric or subatmospheric pressure, although operation at superatmospheric pressure is preferred.

As indicated above maleic anhydride catalysts containing the mixed oxides of vanadium and phosphorus can be activated by contacting the catalyst in the absence of molecular oxygen with gaseous hydrocarbons or reducing agents such as hydrogen or hydrogen sulfide. Such activation is only suitable for fixed bed catalyst forms, but as explained above, cannot readily be utilized as an in situ activation for fluidizable catalysts in a fluid bed reactor.

In the process of the present invention, fluidizable catalysts containing the mixed oxides of vanadium and phosphorus are activated by contacting the catalyst with oxygen and a reducing gas at least partially combustible with oxygen at an elevated temperature sufficient to cause such combustion, in a molar ratio of reducing gas to oxygen above the stoichiometric ratio required for complete combustion and indeed in a ratio of reducing gas to oxygen of from 10:1 to 1:3.

Suitable reducing gases include the hydrocarbon being utilized as a reactant to provide maleic anhydride, such as n-butane, n-butenes, and butadiene although other hydrocarbons are also suitable.

Such other hydrocarbons may have up to 10 carbon atoms and may include methane, ethane, propane, isobutane, isobutylene, pentane, hexane and benzene. Other suitable reducing agents include hydrogen, ammonia, carbon monoxide and hydrogen sulfide.

Oxygen may be added as air or synthetic streams containing molecular oxygen may be utilized. Inert gases may be added to the activating feed, examples of such gases being nitrogen, argon, carbon dioxide and steam.

The molar ratio of reducing gas to oxygen in the activation feed stream is 10:1 to 1:3, preferably 5:1 to 1:2. If air is used as the source of oxygen, the molar ratio of reducing gas to air is 2:1 to 1:15 preferably 1:1 to 1:10. Inert gases in addition to those provided by air in the activation feed, may be added according to the process of the present invention. The amount of total inert gas to reducing gas may be between 0:1 to 50:1 or greater, although preferably the amount of inert gas added to the activation feed, in addition to the inerts provided by air, are added in the molar ratio of inerts to reducing gas of 3:1 to 30:1.

The activation procedure should be carried out at a temperature high enough to cause and sustain at least partial combustion of the reducing gas. The temperature required varies for different catalyst formulations, but can be determined by simple experimentation. Generally, the activation procedure is carried out at temperatures between 400°C and 550°C.

The time required for activation depends in part upon the degree of activation required and the temperature at which the activation is carried out. In general, higher activation temperatures and longer activation time, independently contribute to a more activated form of the catalyst. Activation may take place at subatmospheric, atmospheric or superatmospheric pressures.

Activating conditions can be reached by various methods. The change to and from activating temperatures may be accomplished under reaction feeds, activating feeds, or an inert gas.

The activation procedure according to the process of the present invention may be carried out in the fluid bed reactor at any time in the catalysts' life that activity is desired to be increased. It is preferred, however, that the activation procedure be carried out with catalyst that has been operated to produce maleic anhydride from hydrocarbon. That is, the activation procedure seems to have a more beneficial effect upon catalyst in which active sites of catalytic activity have been established by working to catalyze the reaction of hydrocarbon, preferably n-butane, to maleic anhydride, in contrast to catalyst which has not yet been subjected to normal maleic anhydride producing conditions.

The products of the activation procedure are essentially the combustion products CO and $CO_2$, when hydrocarbons are utilized as a reducing gas. Even when n-butane is utilized as the reducing gas, during activation there is little or no maleic anhydride production. There is some hydrocarbon breakthrough, however, and this can be either recycled or disposed of in a catalytic incinerator, without permitting an explosive mixture of oxygen and heated hydrocarbon to form.

The following examples illustrate the invention:—

## Example 1

A catalyst containing the mixed oxides of vanadium and phosphorus, having a phosphorus to vanadium ratio of 1.2:1 were prepared as described in EP—A—0056901 (Application No: 81305882.3). The fluidizable catalyst was used to produce maleic anhydride from n-butane in a 440 cc fluid bed reactor consisting of a 51 cm length of stainless steel tubing having an outer diameter of 3.8 cm, having a stainless steel sparger at the bottom of the tube to act as a gas (air) distributor with an axial 0.64 cm outer diameter thermowell and a separate hydrocarbon inlet at the bottom of the tube. The reactor was fitted with internal gas redistributing baffles. Gas preheating and reactor temperature control was accomplished by placement of the reactor unit in a thermostatic fluidized sand bath.

Flasks for receiving the product maleic anhydride were air cooled, and tail gases were routed to a gas chromatograph for analysis. Reaction conditions and results of the tests run are described in the Table below. The throughput of hydrocarbon feed in the production of maleic anhydride, or the working rate imposed upon the catalyst can be described as WWH, or weight of feed/weight of catalyst/hour, being 0.05 WWH.

After the catalyst had been run for 200 hours, achieving a molar yield of 51.4%, the activation feed of 1 mole butane to 1 mole air to 3 moles additional nitrogen was used to fluidize the catalyst bed at temperature of 480°C for 19.4 hours. After the activation procedure, reaction feeds of 1 mole of butane to 30 moles of air were resumed resulting in an increase of activity demonstrated by a 55.6% yield to maleic anhydride. Even after the reaction temperature was dropped 6°C from the post activation run temperature of 421°C the activity of the catalyst remained excellent, with a 55.0% yield of maleic anhydride. Reaction and activation conditions and results for example 1 and the following examples are contained in the Table below.

## Examples 2—5

Catalysts were prepared according to the procedure of Example 1. The catalysts were used to produce maleic anhydride by n-butane oxidation, and were subjected to the activation process of the present invention under varied times, temperatures, and activation feeds as set out in the Table below. After the activation had been carried out in each example, the catalysts exhibited a substantial increase in activity, demonstrated by a sizable increase in the yield of maleic anhydride. In example 5, the catalyst underwent two successive activations, with a substantial increase in activity resulting after both activation procedures.

## 0 084 706

### Examples 6—8

Vanadium, phosphorus mixed oxide containing catalysts were prepared by methods designed to produce catalytic material having lower activity than the catalysts prepared above, to determine whether the activation procedure of the present invention was useful in increasing the activity of low conversion catalysts. As reported in the Table below, in each Example, the activation procedure resulted in a substantial increase in catalytic activity. These catalysts were tested according to the run procedure of Example 1.

The activation procedure may be carried out while the reactor is "on line" or operating, by changing the reaction feed to the activation feed and either maintaining or increasing temperature. It is also within the scope of the present invention that a slip stream be provided from the reactor to permit catalyst to be continuously withdrawn, subjected to the activation procedure in a continuous loop and reintroduced into the main reactor continuously while the reactor continues in operation. Such a procedure would permit the maintenance of a high level of activity in the reactor catalyst bed by continuously introducing freshly activated catalyst to the main catalyst bed.

Activation/Operation of Vanadium Phosphorus Mixed Oxide Catalysts for
N-Butane Oxidation to Maleic Anhydride

Run Feed = 30 Air/1 HC
WWH = 0.05

| Example No. Recovery Runs | | Activation | | |
| --- | --- | --- | --- | --- |
| | | Feed HC/Air/$N_2$ | Temperature °C | Time Hours |
| 1. | Before | 1/1/3 | 480 | 19.4 |
| | After | | | |
| 2. | Before | 1/10/15 | 460—472 | 19 |
| | After | | | |
| 3. | Before | 1/1/3 | 420 | 18 |
| | After | | | |
| 4. | Before | 1/1/3 | 500 | 2 |
| | After | | | |

5

| Table (continued) Example No. Recovery Runs | Run Temperature °C | % Total Conversion | Maleic Anhydride % Yield | % Selectivity |
|---|---|---|---|---|
| 1. Before | 422 | 90.4 | 51.4 | 56.9 |
| 1. After | 421 | 94.2 | 55.6 | 59.1 |
| 2. Before | 420 | 87.9 | 49.2 | 56.0 |
| 2. After | 419. | 92.1 | 56.2 | 61 |
| 3. Before | 421 | 86.5 | 53.2 | 61.5 |
| 3. After | 420 | 90.8 | 57.7 | 63.5 |
| 4. Before | 420 | 87.6 | 50.8 | 58.0 |
| 4. After | 400 | 85.9 | 57.1 | 66.5 |

HC = n-butane

| Example No. Recovery Runs | Activation | | |
|---|---|---|---|
| | Feed HC/Air/N$_2$ | Temperature °C | Time Hours |
| 5. Before 1st | 1/1/3 | 460 | 19.2 |
| 5. After 1st Before 2nd | 1/1/3 | 480 | 15.5 |
| 6. Before / After | 1/1/3 | 460 | 5.2 |
| 7. Before / After | 1/10/5.7 | 460 | 14.6 |
| 8. Before / After | 1/1/3 | 500 | 16.5 |

Table (continued)

| Example No. Recovery Runs | Run Temperature °C | % Total Conversion | Maleic Anhydride | |
|---|---|---|---|---|
| | | | % Yield | % Selectivity |
| **5.** Before 1st | 419 | 86.0 | 52.1 | 60.5 |
| After 1st | | | | |
| Before 2nd | 419 | 89.4 | 55.7 | 62.5 |
| After 2nd | 420 | 92.4 | 58.5 | 63.5 |
| **6.** Before | 421 | 60.3 | 41.2 | 70.2 |
| After | 421 | 66.9 | 46.9 | 70.1 |
| **7.** Before | 421 | 61.8 | 38.7 | 62.6 |
| After | 419 | 68.3 | 43.7 | 64.1 |
| **8.** Before | 421 | 50.7 | 29.5 | 58.2 |
| After | 420 | 70.6 | 47.0 | 66.6 |

## Claims

1. A process for activating a fluid bed catalyst by contacting a fluidized catalyst containing the mixed oxides of vanadium and phosphorus with oxygen and a reducing gas at least partially combustible with oxygen at an elevated temperature sufficient to cause combustion, wherein the molar ratio of reducing gas to oxygen is above the stoichiometric ratio required for complete combustion of the reducing gas characterized in that a molar ratio of reducing gas to oxygen from 10:1 to 1:3 is employed.

2. A process as claimed in claim 1 characterized in that a ratio of reducing gas to oxygen of from 5:1 to 1:2 is employed.

3. A process as claimed in claim 1 or 2 characterized in that the source, of oxygen, is air.

4. A process as claimed in any of claims 1 to 3 characterized in that the catalyst is additionally contacted with an inert gas, preferably in addition to that in any air feed.

5. A process as claimed in any of claims 1 to 4 characterized in that the temperature is within a range of 400°C to 550°C.

6. A process as claimed in any of claims 1 to 5 characterized in that the reducing gas is selected from $H_2$, $H_2S$, CO, hydrocarbons having from 1 to about 10 carbon atoms, and mixtures thereof.

7. A process as claimed in any of claims 1 to 6 characterized in that the catalyst additionally contains a promoter element selected from at least one of an alkali metal, an alkaline earth metal, titanium zirconium, hafnium, niobium, molybdenum, iron, cobalt, nickel, iron, cobalt, copper, zinc, cadmium, cerium, rare earths, uranium and mixtures thereof.

8. A process as claimed in any of claims 1 to 7 characterized in that phosphorus vanadium ratio in the catalyst is from 0.5:1 to 2:1.

9. A process for the production of maleic anhydride by the oxidation of 4-carbon hydrocarbons with molecular oxygen or an oxygen containing gas in a fluid bed reactor at a reaction temperature of 325°C to 500°C in the presence of a catalyst containing the mixed oxides of vanadium and phosphorus, characterized in that it includes a process step in which the catalyst or a part thereof is subjected to an activation process as claimed in any of claims 1 to 8, preferably utilizing a 4-carbon hydrocarbon as reducing gas.

10. A process as claimed in claim 9 characterized in that catalyst is continuously withdrawn from the reactor, activated by a process as claimed in any of claims 1 to 8, and returned to the reactor.

## Patentansprüche

1. Verfahren zum Aktivieren eines Fließbett-Katalysators, bei dem ein die gemischten Oxide von Vanadium und Phosphor mit Sauerstoff enthaltender Katalysator und ein wenigstens teilweise mit Sauerstoff bei einer für die Verbrennung ausreichenden erhöhten Temperatur brennbares reduzierendes Gas in Kontakt miteinander gebracht werden, wobei das Molekularverhältnis des reduzierenden Gases zum Sauerstoff oberhalb des für die vollständige Verbrennung des reduzierenden Gases erforderlichen stöchiometrischen Verhältnisses liegt, dadurch gekennzeichnet, daß ein molares Verhältnis des reduzierenden Gases zum Sauerstoff von 10:1 bis 1:3 angewendet wird.

2. Verfahren nach Anspruch 1, dadurch gekennzeichnet, daß ein Verhältnis des reduzierenden Gases zum Sauerstoff von 5:1 bis 1:2 angewendet wird.

3. Verfahren nach Anspruch 1 oder 2, dadurch gekennzeichnet, daß die Sauerstoffquelle Luft ist.

4. Verfahren nach einem der Ansprüche 1 bis 3, dadurch gekennzeichnet, daß der Katalysator zusätzlich mit einem inerten Gas, vorzugsweise zusätzlich zu dem, das in jeder Frischluft enthalten ist, in Kontakt gebracht wird.

5. Verfahren nach einem der Ansprüche 1 bis 4, dadurch gekennzeichnet, daß die Temperatur in einem Bereich von 400°C bis 550°C liegt.

6. Verfahren nach einem der Ansprüche 1 bis 5, dadurch gekennzeichnet, daß das reduzierende Gas aus $H_2$, $H_2S$, CO, Kohlenwasserstoffe mit 1 bis 10 Kohlenstoffatomen und Gemischen daraus ausgewählt wird.

7. Verfahren nach einem der Ansprüche 1 bis 6, dadurch gekennzeichnet, daß der Katalysator zusätzlich ein Beschleunigerelement enthält, das aus wenigstens einem von Alkalimetall, einem alkaliner Erdmetall, Titan, Zirkon, Hafnium, Niob, Molybden, Eisen, Kobalt, Nickel, Kupfer, Zink, Kadmium, Cer, seltene Erden, Uran und Gemische derselben besteht.

8. Verfahren nach einem der Ansprüche 1 bis 7, dadurch gekennzeichnet, daß das Phosphor-Vanadium-Verhältnis im Katalysator von 0,5:1 bis 2:1 beträgt.

9. Verfahren zum Herstellen von Maleinsäureanhydrid durch Oxidation von vier C-Atome aufweisenden Kohlenwasserstoffen mit molekularem Sauerstoff oder einem Sauerstoff enthaltenden Gas in einem Fließbettreaktor bei einer Reaktionstemperatur von 325°C bis 500°C in Gegenwart eines Katalysators, der die gemischten Oxide von Vanadium und Phosphor enthält, dadurch gekennzeichnet, daß es einen Verfahrensschritt einschließt, in welchem der Katalysator oder ein Teil desselben einem Aktivierungsverfahren unterworfen wird, wie es in einem der Ansprüche 1 bis 8 beansprucht ist, wobei vorzugsweise ein vier C-Atome aufweisender Kohlenwasserstoff als reduzierendes Gas verwendet wird.

10. Verfahren nach Anspruch 9, dadurch gekennzeichnet, daß der Katalysator kontinuierlich vom Reaktor abgezogen, durch ein Verfahren wie in einem der Ansprüche 1 bis 8 beansprucht, aktiviert und in den Reaktor zurückgeleitet wird.

## Revendications

1. Procédé d'activation d'un catayseur en lit fluidisé en mettant en contact un catalyseur fluidisé contenant des oxydes mixtes de vanadium et de phosphore avec de l'oxygène et un gaz réducteur ou moins partiellement combustible avec l'oxgene à une température élevée suffisante pour provoquer la combustion, dans lequel le rapport molaire due gaz réducteur à l'oxygène est supérieur au rapport stoechiométrique nécessaire pour une combustion complète du gaz réducteur, caractérisé par le fait qu'on utilise un rapport molaire du gaz réducteur à l'oxygéne de 10:1 à 1:3.

2. Procédé selon la revendication 1, caractérisé par le fait qu'on utilise un rapport du gaz réducteur à l'oxygène de 5:1 à 1:2.

3. Procédé selon la revendication 1 ou 2, caractérisé par le fait que la source d'oxygène est l'air.

4. Procédé selon l'une quelconque des revendications 1 à 3, caractérisé par le fait que le catalyseur est en outre mis en contact avec un gaz inerte, de préférence en plus de celui présent dans tout courant d'alimentation d'air.

5. Procédé selon l'une quelconque des revendications 1 à 4, caractérisé par le fait que la température est dans un intervalle de 400 à 550°C.

6. Procédé selon l'une quelconque des revendications 1 à 5, caractérisé par le fait que le gaz réducteur est choisi parmi $H_2$, $H_2S$, CO, des hydrocarbures ayant de 1 à environ 10 atomes de carbone, et des mélanges de ceux-ci.

7. Procédé selon l'une quelconque des revendications 1 à 6, caractérisé par le fait que le catalyseur contient en outre un élément promoteur choisi parmi au moins un des suivants: métal alcalin, métal alcalino-terreux, titane, zirconium, hafnium, niobium, molybdène, fer, cobalt, nickel, cuivre, zinc, cadmium, cérium, terres rares, uranium et mélanges de ceux-ci.

8. Procédé selon l'une quelconque des revendications 1 à 7, caractérisé par le fait que le rapport du phosphore/vanadium dans le catalyseur est de 0,5:1 à 2:1.

9. Procédé de production d'anhydride maléique par oxydation d'hydrocarbures en $C_4$ avec de l'oxygène moléculaire ou un gaz contenant de l'oxygène dans un réacteur à lit fluidisé à une température de réaction de 325°C à 500°C en présence d'un catalyseur contenant les oxydes mixtes de vanadium et de phosphore, caractérisé par le fait qu'il comprend un stade de procédé dans lequel le catalyseur ou une partie de celui-ci est soumis à une opération d'activation selon l'une quelconque des revendications 1 à 8 de préférence en utilisant un hyrocarbure en $C_4$ comme gaz réducteur.

8

10. Procédé selon la revendication 9, caractérisé par le fait que le catalyseur est soutiré en continu du réacteur, activé par un procédé selon l'une quelconque des revendications 1 à 8, et renovoyé dans le réacteur.